Europäisches Patentamt

European Patent Office

Office européen des brevets

(11) Veröffentlichungsnummer: **0 292 915**
**A1**

(12) **EUROPÄISCHE PATENTANMELDUNG**

(21) Anmeldenummer: **88108257.2**

(22) Anmeldetag: **24.05.88**

(51) Int. Cl.⁴: **A61F 5/47**

(30) Priorität: **25.05.87 DE 3717538**

(43) Veröffentlichungstag der Anmeldung:
**30.11.88 Patentblatt 88/48**

(84) Benannte Vertragsstaaten:
**AT BE CH DE ES FR GB IT LI NL SE**

(71) Anmelder: **Tauber, Peter F.**

**D-5900 Siegen(DE)**

Anmelder: **KETASA AG**

**CH-4106 Therwil(CH)**

(72) Erfinder: **Keck, H., Dr.**
**13 Blauenstrasse**
**CH-4106 Therwil/Baselland(CH)**
Erfinder: **Tauber, Peter F.**
**St. Marien-Krankenhaus, Kampenstrasse**
**D-5900 Siegen(DE)**

(74) Vertreter: **WILHELMS, KILIAN & PARTNER**
**Patentanwälte**
**Eduard-Schmid-Strasse 2**
**D-8000 München 90(DE)**

(54) **Empfängnisverhütende Vorrichtung.**

㊻ Empfängnisverhütende Vorrichtung aus elastischem Material zum Einführen in den Uterus, die mit einem kontrazeptiven Mittel versehen ist, wobei die empfängnisverhütende Vorrichtung (1) im wesentlichen herzförmig ausgebildet ist und wobei ein kontrazeptives Mittel (4, 4') auf zwei Bögen (1a, 1a') der empfängnisverhütenden Vorrichtung (1) in Tubeneingangsnähe vorgesehen ist.

FIGUR 1

EP 0 292 915 A1

## Empfängnisverhütende Vorrichtung

Die Erfindung betrifft eine emfängnisverhütende Vorrichtung aus elastischem Material zum Einführen in den Uterus, die mit einem kontrazeptiven Mittel versehen ist.

In der DE-OS 22 49 169 wird eine Vorrichtung dieser Gattung beschrieben. Diese bekannte Vorrichtung ist aufgrund ihrer flachen, schildartigen Form wenig elastisch und gibt deshalb bei Uteruskontraktionen nicht nach, wodurch Verletzungen der Uterus-Schleimhaut hervorgerufen und Schmerzen verursacht werden. Nachteilig an dieser bekannten Vorrichtung ist außerdem, daß sie von dem Einführstab nach dem Einführen in die Gebärmutter durch verhältnismäßig komplizierte Bewegungen gelöst werden muß.

Eine andere empfängnisverhütende Vorrichtung wird in der DE-PS 25 05 104 beschrieben. Bei dieser Vorrichtung sind zwei biegsame Arme am Ende eines Stils angebracht, um den eine Kupferdrahtspirale gewickelt ist. Einerseits können durch die Arme beim Herausziehen Verletzungen hervorgerufen werden, andererseitsentfaltet die Kupferdrahtspirale ihre Wirkung in der Mitte des Uterus und nicht an den für die Empfängnisverhütung wichtigen Stellen, d.h. den Tubeneingängen.

Nachteilig an beiden Vorrichtungen ist darüberhinaus,daß sie mittels eines an ihnen befestigten Fadens,der aus dem Uterus herausragt, entfernt werden müssen. Es ist erwiesen, daß Bakterien an diesem Faden in den Uterus gelangen und dort bösartige Entzündungen bis zu den Eileitern hervorrufen.

Der Erfindung liegt daher die Aufgabe zugrunde, eine empfängnisverhütende Vorrichtung zur Verfügung zu stellen, die bei leichter Einführbarkeit aufgrund ihrer elastischen Eigenschaften gut verträglich ist, die bessere empfängnisverhütende Eigenschaften besitzt und bei der die Wahrscheinlichkeit, daß es durch von außen eintretende Bakterien zu Infektionen kommt, vermindert wird.

Die erfindungsgemäße Aufgabe wird durch die kennzeichnenden Merkmale des Hauptanspruchs gelöst.

Weitere vorteilhafte Ausgestaltungen der Erfindung ergeben sich aus den nachfolgenden Ansprüchen 2 bis 10.

Die leichte Verformbarkeit sowie das gute Rückstellvermögen nach Verformung der erfindungsgemäßen Vorrichtung wird durch die gelenkige Verbindung der beiden Hälften gewährleistet. Diese Flexibilität wird noch erhöht, weil die beiden bogenförmigen Hälften im oberen Bereich der empfängnisverhütenden Vorrichtung nicht miteinander verbunden, sondern gegeneinander beweglich sind. Durch die auf die Mittelachsen aufschiebbare Einführungsvorrichtung kann die erfindungsgemäße Vorrichtung leicht eingeführt werden. Ein kontrazeptives Mittel ist an den Stellen der Vorrichtung angebracht, die nach dem Einführen der Vorrichtung nahe den Tubeneingängen liegen und so gleichsam eine "Spermatozoenfalle" bilden. Erfindungsgemäß wird das Element zum Entfernen der empfängnisverhütenden Vorrichtung nur dann in diese eingehängt, wenn sie tatsächlich entfernt werden soll; das Eindringen von Bakterien über dieses Element wird also verdert. Die dünne Kupferschicht, mit der die ganze Oberfläche der empfängnisverhütenden Vorrichtung bedeckt ist, ist besonders aktiv und übt nach der Einführung eine Art Initialwirkung aus , noch bevor die Wirkung der Kupferdrahtspiralen voll eingesetzt hat.

Die Erfindung wird anhand der Figuren 1 bis 3 näher erläutert.

Fig. 1 ist die Draufsicht einer Ausführungsform der erfindungsgemäßen empfängnisverhütenden Vorrichtung.

Fig. 2 ist die Darstellung der empfängnisverhütenden Vorrichtung mit dem erfindungsgemäßen Element zur Entfernung der Vorrichtung.

Fig. 3 ist die Darstellung der empfängnisverhütenden Vorrichtung mit der Einführungsvorrichtung.

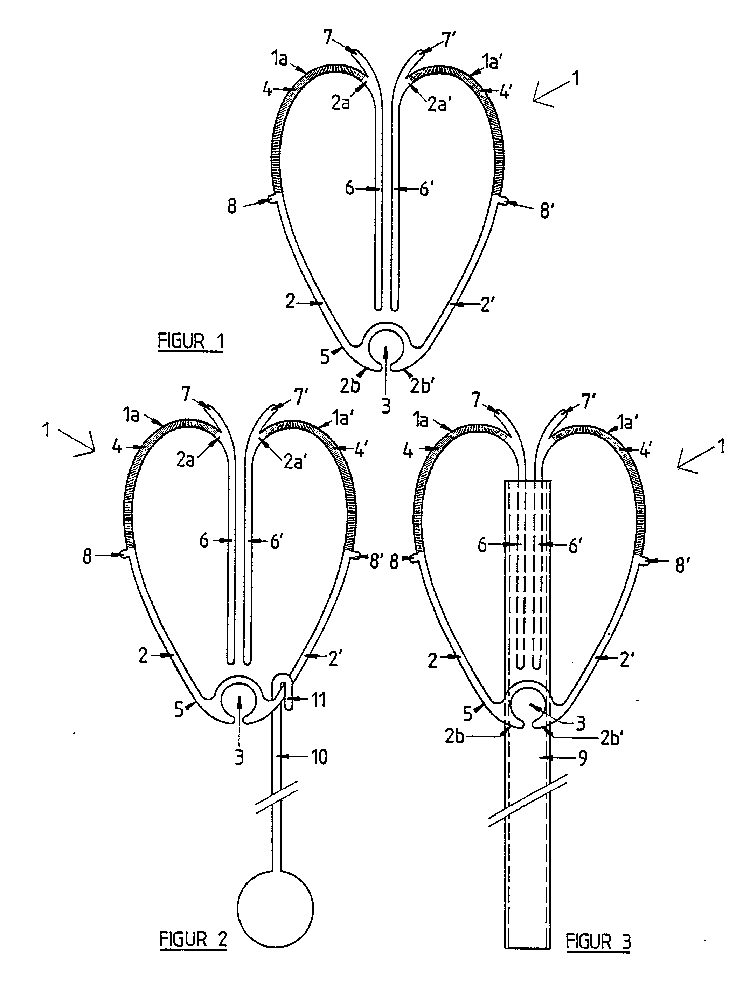

Gemäß Fig. 1 ist die empfängnisverhütende Vorrichtung 1 im Wesentlichen herzförmig ausgebildet, wobei ihre beiden Hälften 2, 2' an ihren einen Enden 2b, 2b' durch ein gelenkiges Teil 3 miteinander verbunden sind. Vorzugsweise ist das gelenkige Teil 3 in Form einer elastischen, bogenförmigen Verbindung 1 ausgebildet, die zur Innenseite der empfängnisverhütenden Vorrichtung weist. An den anderen Enden 2a, 2a' der beiden Hälften 2, 2' ist jeweils eine Mittelachse 6, 6' angebracht, wobei die Mittelachsen 6, 6' vorzugsweise parallel und mit geringem Abstand zueinander angeordnet sind.

Auf den Bögen 1a,1a' der empfängnisverhütenden Vorrichtung 1 sind Verhütungsmittel 4, 4', vorzugsweise Kupferdrahtspiralen angebracht, die jeweils durch Noppen 7, 8 und 7', 8' in Stellung gehalten werden. Die Kupferdrahtspiralen 4, 4' haben eine Oberfläche von 200 bis 800 mm², vorzugsweise von 400 bis 600 mm².

Die empfängnisverhütende Vorrichtung besteht aus einem elastischen Material, vorzugsweise besteht sie aus Polyethylen, Ethylen/vinylacetat-Copolymeren oder Polyamid, Die ganze Oberflache der Vorrichtung ist vorzugsweise mit einer dünnen Kupferschicht 5 bedeckt, die vorzugsweise im Hochvakuum aufgedampft wird.

Gemäß Fig. 2 wird die empfängnisverhütende Vorrichtung mit einem Element 10 zum Entfernen der Vorrichtung 1 aus dem Uterus herausgezogen, wobei sie eine längliche Form annimmt. Das Element 10 ist vorzugsweise ein Stab, der endseitig mit einem U-förmigen Haken 11 zum Einhängen in die Vorrichtung 1 versehen ist.

Gemäß Fig. 3 ist die Einführungsvorrichtung 9 vorzugsweise als röhrenförmige Hülse ausgebildet. Diese Hülse 9 wird über beide Mittelachsen 6, 6' geschoben; die empfängnisverhütende Vorrichtung 1 kann auf diese Weise leicht in die Gebärmutter eingeführt werden.

## Ansprüche

1. Empfängnisverhütende Vorrichtung aus elastischem Material zum Einführen in den Uterus, die mit einem kontrazeptiven Mittel versehen ist, dadurch **gekennzeichnet,** daß die empfängnisverhütende Vorrichtung (1) im wesentlichen herzförmig ausgebildet ist, wobei ein kontrazeptives Mittel (4, 4') auf zwei Bögen 1a, 1a') der empfängnisverhütenden Vorrichtung (1) in Tubeneingangsnähe vorgesehen ist.

2. Empfängnisverhütende Vorrichtung nach Anspruch 1, dadurch **gekennzeichnet,** daß ein kontrazeptives Mittel (4, 4') als um die Bögen (1a, 1a') gewickelte Kupferdrahtspiralen ausgebildet ist.

3. Empfängnisverhütende Vorrichtung nach Anspruch 1 und/oder Anspruch 2, dadurch **gekennzeichnet,** daß die beiden Hälften (2, 2') an ihren Enden (2b, 2b') durch ein gelenkiges Teil (3) verbunden werden.

4. Empfängnisverhütende Vorrichtung nach Anspruch 3, dadurch **gekennzeichnet,** daß das gelenkige Teil (3) zur Innenseite der empfängnisverhütenden Vorrichtung (1) weist und elastisch und bogenförmig ausgebildet ist.

5. Empfängnisverhütende Vorrichtung nach mindestens einem der vorhergehenden Ansprüche, dadurch **gekennzeichnet,** daß an den anderen Enden (2a, 2a') der beiden Hälften (2, 2') zwei Mittelachsen(6) zum Aufschieben einer Einführungsvorrichtung (9) angeordnet sind.

6. Empfängnisverhütende Vorrichtung nach mindestens einem der vorhergehenden Ansprüche, dadurch **gekennzeichnet,** daß die gesamte Oberfläche der empfängnisverhütenden Vorrichtung (1) mit einem zweiten kontrazeptiven Mittel in Form einer dünnen Kupferschicht (5) bedeckt ist.

7. Empfängnisverhütende Vorrichtung nach mindestens einem der vorhergehenden Ansprüche, dadurch **gekennzeichnet,** daß ein Element (10) zum Entfernen der empfängnisverhütenden Vorrichtung (1) reversibel mit dieser verbunden ist.

8. Empfängnisverhütende Vorrichtung nach mindestens einem der vorhergehenden Ansprüche, dadurch **gekennzeichnet,** daß das Element (10) ein endseitig mit einem U-förmigen Haken (11) versehener Stab ist.

9. Empfängnisverhütende Vorrichtung nach mindestens einem der vorhergehenden Ansprüche, dadurch **gekennzeichnet,** daß sie aus weichem, flexiblen Kunststoff besteht.

10. Empfängnisverhütende Vorrichtung nach mindestens einem der vorhergehenden Ansprüche, dadurch **gekennzeichnet,** daß sie aus Polyethylen, Ethylen/Vinylacetat-Copolymeren oder Polyamid besteht.

FIGUR 1

FIGUR 2

FIGUR 3

**Europäisches Patentamt**

**EUROPÄISCHER RECHERCHENBERICHT**

Nummer der Anmeldung

| Kategorie | EINSCHLÄGIGE DOKUMENTE | | EP 88108257.2 |
|---|---|---|---|
| | Kennzeichnung des Dokuments mit Angabe, soweit erforderlich, der maßgeblichen Teile | Betrifft Anspruch | KLASSIFIKATION DER ANMELDUNG (Int. Cl.4) |
| A | DE - A1 - 2 841 373 (HASSON)<br>* Seite 13, Zeile 17 - Seite 16, Zeile 27; Fig. 1 *<br>-- | 1,6,9,10 | A 61 F 5/47 |
| D,A | DE - C2 - 2 505 104 (MULTILAN)<br>* Spalte 1, Zeilen 34-45; Spalte 2, Zeile 47 - Spalte 3, Zeile 10; Fig. 1 *<br>-- | 1,2 | |
| A | US - A - 4 200 091 (DEL CONTE)<br>* Zusammenfassung; Spalte 1, Zeile 55 - Spalte 2, Zeile 18; Spalte 3, Zeilen 12-16; Fig. 1 *<br>-- | 1 | |
| A | DE - A - 2 251 383 (A.H.ROBINS)<br>* Seite 19, Zeile 7 - Seite 20, Zeile 4 *<br>-- | 1,6,9,10 | RECHERCHIERTE SACHGEBIETE (Int. Cl.4) |
| D,A | DE - A - 2 249 169 (A.H.ROBINS)<br>* Seite 6, Zeile 17 - Seite 10, Zeile 20; Fig. 1 *.<br>---- | 1,5 | A 61 F |

Der vorliegende Recherchenbericht wurde für alle Patentansprüche erstellt.

| Recherchenort | Abschlußdatum der Recherche | Prüfer |
|---|---|---|
| WIEN | 20-09-1988 | BRUNNER |